# FASCICULE DE BREVET EUROPEEN

(11) **EP 2 007 882 B1**
(45) Date de publication et mention de la délivrance du brevet: **01.09.2010**
(21) Numéro de dépôt: 07727050.2
(22) Date de dépôt: 19.03.2007
(51) Int. Cl.: C12N 5/10, C07K 14/465

(54) **PROCEDE DE PREPARATION DE CELLULES AVIAIRES DIFFERENCIEES ET GENES IMPLIQUES DANS LE MAINTIEN DE LA PLURIPOTENCE**
VERFAHREN ZUR HERSTELLUNG DIFFERENZIERTER VOGELZELLEN UND AN DER AUFRECHTERHALTUNG DER PLURIPOTENZ BETEILIGTER GENE
METHOD FOR PREPARING DIFFERENTIATED AVIAN CELLS AND GENES INVOLVED IN MAINTAINING PLURIPOTENCY

(30) Priorité: 24.03.2006 FR 0602597
(43) Date de publication de la demande: 31.12.2008
(60) Demande divisionnaire: 10170872.5
(73) Titulaire: INSTITUT NATIONAL DE LA RECHERCHE AGRONOMIQUE, 75007 Paris (FR); CENTRE NATIONAL DE LA RECHERCHE SCIENTIFIQUE (CNRS), 75016 Paris (FR); ENS - Ecole Normale Supérieure de Lyon, 69007 Lyon (FR)
(72) Inventeur: PAIN, Bertrand, F-63100 Clermont-Ferrand (FR); LAVIAL, Fabrice, F-42100 Saint Etienne (FR); SAMARUT, Jacques, F-69003 Lyon (FR)
(74) Mandataire: Tetaz, Franck Claude Edouard
(86) Numéro de dépôt international: PCT/EP2007/052572
(87) Numéro de publication internationale: WO 2007/110341

(56) Documents cités:
- WO-A-96/12793
- HOUGH SHELLEY R ET AL: "Differentiation of mouse embryonic stem cells after RNA interference-mediated silencing of OCT4 and Nanog." STEM CELLS (DAYTON, OHIO) JUN 2006, vol. 24, no. 6, juin 2006 (2006-06), pages 1467-1475, XP009073491 ISSN: 1066-5099
- ZAEHRES HOLM ET AL: "High-efficiency RNA interference in human embryonic stem cells" STEM CELLS (MIAMISBURG), vol. 23, no. 3, mars 2005 (2005-03), pages 299-305, XP002402498 ISSN: 1066-5099
- VELKEY J M ET AL: "OCT4 RNA INTERFERENCE INDUCES TROPHECTODERM DIFFERENTIATION IN MOUSE EMBRYONIC STEM CELLS" GENESIS: THE JOURNAL OF GENETICS AND DEVELOPMENT, WILEY-LISS, NEW YORK, NY,, US, vol. 37, no. 1, septembre 2003 (2003-09), pages 18-24, XP008035096 ISSN: 1526-954X
- SATO FUMINORI ET AL: "Application of RNA interference to chicken embryos using small interfering RNA" JOURNAL OF EXPERIMENTAL ZOOLOGY, vol. 301A, no. 10, 1 octobre 2004 (2004-10-01), pages 820-827, XP002402499 ISSN: 0022-104X
- PAIN B ET AL: "Long-term in vitro culture and characterisation of avian embryonic stem cells with multiple morphogenetic potentialities" DEVELOPMENT, COMPANY OF BIOLOGISTS, CAMBRIDGE,, GB, vol. 122, no. 8, 1996, pages 2339-2348, XP002164729 ISSN: 0950-1991
- HAN JAE YONG ET AL: "Gene expression profiling of chicken primordial germ cell ESTs" BMC GENOMICS, BIOMED CENTRAL, LONDON, GB, vol. 7, no. 1, 30 août 2006 (2006-08-30), page 220, XP021014598 ISSN: 1471-2164 & DATABASE EMBL [Online] 28 juin 2006 (2006-06-28), "gPGC_EST00245 Embryonic gonadal PGC cDNA Library Gallus gallus cDNA 5', mRNA sequence." extrait de EBI accession no. EMBL:DR410403 Database accession no. DR410403

## Description

La présente invention concerne un procédé de préparation de cellules aviaires différenciées, à partir de cellules souches en culture. Des gènes impliqués dans le maintien de la pluripotence des cellules souches aviaires ont été identifiés et clonés. En inhibant l'expression de ces gènes dans les cellules souches, celles-ci perdent leurs caractéristiques de pluripotence et s'engagent dans une voie de différenciation. Ces cellules différenciées obtenues *in vitro* peuvent servir de cellules hôtes pour des pathogènes, notamment des virus, et être ainsi utilisées pour la production de vaccins anti-viraux.

Une cellule souche est une cellule pluripotente ou multipotente d'origine embryonnaire ou adulte qui présente une capacité d'auto-renouvellement. En d'autres termes, une cellule souche est une cellule non cancéreuse capable de se diviser indéfiniment en culture et de donner une cellule fille présentant la même capacité de prolifération et de différenciation que la cellule mère dont elle est issue, et qui est capable de donner naissance à des cellules différenciées.

Les cellules souches embryonnaires de poulet (CESC pour Chicken Embryonic Stem Cells) ont été isolées par la mise en culture de cellules de blastodermes de poulet au stade X (Pain et al., 1996; demande de brevet N° FR 94/12598). Ces cellules CESC présentent toutes les caractéristiques de cellules souches embryonnaires (ESC). Un milieu de culture permettant de maintenir le caractère pluripotent de ces cellules d'oiseaux a fait l'objet de la demande de brevet WO 96/12793. La plupart des caractéristiques de ces cellules sont décrites dans la publication Lavial et al. (soumis).

Une des propriétés des cellules souches est leur capacité à donner des cellules différenciées à la fois *in vitro* et *in vivo.* L'obtention de ces cellules différenciées à partir d'une population homogène de précurseurs est réalisée par la modification des conditions de culture. En effet, les cellules CESC ne demeurent en prolifération dans un état non différencié que dans des conditions définies de culture *in vitro,* en présence de facteurs de croissance et de cytokines ainsi que de 'feeder' inactivé. Dès que le milieu est appauvri, l'équilibre de prolifération est modifié et la cellule s'engage en différenciation. Un des avantages de l'obtention de cellules différenciées *in vitro* à partir des cellules CESC est la très large palette de phénotypes qui peuvent être générés à partir de ces cellules. En effet, les cellules CESC présentent des propriétés de pluripotence ; elles peuvent se différencier en tous les lignages définis au niveau embryologique, soit en dérivés mésodermiques, ectodermiques ou endodermiques. Cette propriété de pluripotence, spécifique d'une cellule souche, n'est pas présente dans les cellules primaires d'un tissu qui sont déjà engagées dans une voie de différenciation. Cette propriété est maximale dans les cellules souches embryonnaires et est présente mais de façon plus restreinte au niveau des cellules souches adultes tissulaires, qui sont multipotentes. Ces dernières ne peuvent se différencier que dans des voies de différenciation appartenant à un seul et même lignage.

Les processus de re-programmation nucléaire permettent à une cellule différenciée de retrouver une plasticité de différenciation. Ces mécanismes commencent à être décryptés au niveau moléculaire notamment par la compréhension des modifications épigénétiques qui se produisent au niveau de l'ADN (méthylation sur les ilotsCpG) et /ou de ses composants comme les histones par des processus d'acétylation et de déacétylation, de méthylation et de déméthylation, d'ubiquitination des lysines, de phosphorylation des sérines, d'isommérisation des prolines et dont une des conséquences est de recruter des e de nombreux complexes protéiques qui contrôlent par leur association sur des promoteurs le niveau d'expression de gènes clefs. Des modifications post traductionnelles directes de ces acteurs sont également un élément de régulation supplémentaire.

Différents procédés sont connus par l'homme du métier pour induire ou contrôler la différenciation des cellules souches pluripotentes en cellules différenciées *in vitro.*

Une première approche consiste à ensemencer les cellules dans un milieu de culture contenant peu ou pas de cytokines et facteurs de croissance nécessaires au maintien de la prolifération des cellules. En particulier, l'absence ou la faible concentration d'une des cytokines de la famille ge130 (LIF, IL-6, CNTF, GPA, IL-11, ...) induit un ralentissement de la prolifération et une perte progressive des marqueurs de la pluripotence.

La différenciation des cellules soumises à ce procédé n'est pas homogène ; différents types cellulaires seront obtenus selon les conditions de densité, de sécrétion autocrine et paracrine des cellules, et de leurs relations les unes avec les autres. L'hétérogénéité des cellules obtenues peut être estimée par la détection dans l'ensemble de la culture de la plupart des marqueurs précoces de lignages, comme par exemple les gènes *Brachyury* et *Goosecoid* spécifiques du lignage mésodermique, certains gènes *Pax* et *Sox* spécifiques du lignage neurectodermique, et *Hnf3* spécifique du lignage endodermique.

Une seconde approche consiste à réaliser des corps embryoïdes par ensemencement des cellules dans une boite qui n'est pas traitée pour la culture cellulaire. Les cellules dissociées sont mises en suspension soit dans un grand volume de milieu appauvri (moins de sérum - de 0,5 à 5% par exemple - et une absence de facteurs de croissance et de cytokines spécifiques) et soumis à une agitation lente, soit dans une petit volume de milieu appauvri qui est alors utilisé dans la technique des gouttes pendantes. Différents exemples sont donnés dans les brevets US 5,456,357; US 5,914,268 et US 6,458,589. Un autre conditionnement est la formation des corps embryoïdes directement dans un tube conique (Kurosawa et al., 2003), sur une surface traitée particulière (Konno et al., 2005) ou par encapsulation dans des microbilles d'alginate (Magyar et al., 2001). En empêchant ainsi l'adhésion des cellules et leur polarisation baso-latérale, les cellules souches embryonnaires prolifèrent et adoptent une structure tridimensionnelle qui mime les différents feuillets embryonnaires (Dang et al., 2002). Les types cellulaires ainsi obtenus sont très hétérogènes. De plus on observe une hétérogénéité dans la cinétique d'obtention des différents stades de différenciation.

Une troisième approche est l'utilisation d'inducteurs chimiques de différenciation. Par substance inductrice chimique, on entend toute molécule chimique non peptidique ne s'apparentant pas à un facteur de croissance ou à une cytokine, qu'elle soit d'origine naturelle ou obtenue par synthèse chimique.

Par exemple le DMSO (Diméthylsulfoxide) est utilisé comme un inducteur général qui permet l'obtention de populations différenciées mixtes avec plusieurs types cellulaires dérivés (Dinsmore et al., 1996). L'acide rétinoïque permet, seul ou en combinaison avec l'AMPc par exemple, de façon non exclusive, d'obtenir la différenciation des cellules souches notamment en dérivés mésodermiques, avec l'obtention d'adipocytes dans des conditions de cinétique précises (Dani et al., 1997), de cardiomyocytes, de différentes cellules musculaires caractérisées par une activité contractile et la présence de myosine spécifique (Rohwedel et al., 1994 ; Drab et al., 1997).

La plupart des protocoles qui sont actuellement publiés et qui permettent l'obtention de cellules avec un phénotype particulier à partir de cellules souches embryonnaires de souris associent des inducteurs chimiques et des combinatoires complexes de facteurs de croissance. Des variations dans les cinétiques d'induction et de mise en présence de ces agents permettent de moduler les phénotypes obtenus. Des exemples sont donnés avec la cinétique d'obtention de cellules adipocytaires (Dani et al., 1997), de cellules ostéoblastiques (ZurNieden et al., 2003 ; Philips et al., 2001), et de précurseurs neuronaux présentant différents phénotypes (Fraichard et al., 1995 ; Plachta et al., 2004 ; Glaser and Brustle, 2005).

La reproductibilité de telles approches est parfois difficile et ne permet pas d'obtenir des enrichissements en cellules différenciées satisfaisants pour des applications industrielles, comme la réplication de virus particuliers ou la production de molécules d'intérêt. De plus les processus comprennent de nombreuses étapes, impliquant l'utilisation de milieux de culture bien particuliers et de nombreuses étapes de manipulation des cellules.

Les approches décrites précédemment permettent d'induire la différenciation de cellules souches ; les populations obtenues étant hétérogènes, il est ensuite nécessaire de sélectionner et d'isoler les cellules présentant le phénotype souhaité; pour cela plusieurs techniques sont connues de l'homme du métier. On peut citer de façon non exclusive le tri par trieur de surface suite aux marquages par des anticorps de surface spécifiques, par déplétion ou par enrichissement magnétique.

Une autre approche consiste à utiliser des protocoles d'enrichissement qui font appel à des processus de sélections génétiques positives. Pour cela l'expression d'un gène de sélection d'une drogue (néomycine, hygromycine, zéomycine, blasticidine) ou d'un marqueur phénotypique permettant le tri physique d'une cellule qui l'exprime (protéines fluorescentes comme les GFP sauvages ou modifiées, beta-galactosidase, alcool deshydrogénase) est placé sous la dépendance d'un promoteur tissu-spécifique. Parmi les promoteurs utilisés on peut citer ceux des chaînes de *Myosine* (Muller et al., 2000), ceux des gènes inducteurs de la myogénèse *Mvf5* ou *MyoD,* ceux de la *Nestine* (Keyoung et al., 2001), de *Hb9* (SinghRoy et al., 2005), de promoteurs spécifiques des astrocytes comme la *GFAP* (Benveniste et al., 2005) ou d'autres types neuronaux comme les oligodendrocytes avec les genes *CNP* (cyclic 3' phosphodiesterase) (Glaser et al., 2005; Schmandt et al., 2005).

Une autre approche consiste à sur-exprimer le cDNA d'intérêt capable d'induire la différenciation dans un lignage donné, comme par exemple avec les cDNA du lignage myoblastique (gènes *Pax3, MyoD, Myf5, Myognénine, Mft-4,* ...). Cette sur-expression peut être réalisée à l'aide de vecteurs de sur-expression à insertion aléatoire, mais aussi à l'aide d'une stratégie de knock in dans un locus donné de l'actine ou dans le locus spécifique du lignage, ou encore à l'aide de vecteurs viraux et rétroviraux.

A l'heure actuelle il n'existe donc pas de méthode permettant d'obtenir une population de cellules différenciées *in vitro* qui soient homogènes, sans réaliser ultérieurement une étape de sélection. C'est pourquoi les inventeurs ont mis au point une nouvelle technique permettant d'obtenir une population de cellules différenciées présentant des caractéristiques morphologiques, biochimiques et fonctionnelles homogènes, qui ne nécessite pas d'étape de sélection supplémentaire. Selon l'invention, il est possible d'obtenir une modification morphologique, phénotypique et moléculaire de 80 à 100% des cellules d'un clone induit, c'est-à-dire d'un clone composé de cellules différenciées.

Chez les mammifères, plusieurs gènes contrôlant la pluripotence et les capacités de prolifération des cellules souches ont été identifiés. En particulier le gène *Oct4* (Nichols et al., 1998) est un gène clé exprimé uniquement dans les cellules pluripotentes *in vivo. In vitro, Oct4* est exprimé dans les cellules souches embryonnaires de souris et de primates, ainsi que dans certaines lignées cellulaires tumorales. Le niveau d'expression de *Oct4* dans des cellules souches embryonnaires murines semble contrôler le devenir de ces cellules souches (Niwa et al., 2000).

Le gène *Oct4* appartient à la famille POU des facteurs de transcription à homéodomaine; la protéine produit du gène a une localisation nucléaire et peut se fixer directement sur les éléments de régulation des gènes cibles via son domaine de fixation à l'ADN.

D'un point de vue phylogénétique, il était peu probable qu'un gène de fonction équivalente existe chez les espèces non mammifères. Chez les oiseaux, un tel gène n'avait pu être identifié lors de travaux précédents (Sooden-Karamath & Gibbins, 2001). De plus aucune homologie au niveau génomique n'a été reportée.

Chez les mammifères, les gènes *Nanog* (Chambers et al. 2003: Mitsui et al., 2003; Hart et al; 2004) et Eomes (Russ et aL, 2000, Ginis et al., 2004) ont également été identifiés comme jouant un rôle clé dans le maintien de la pluripotence des cellules souches.

La présente invention propose un procédé de préparation de cellules aviaires différenciées à partir de cellules souches aviaires cultivées dans un milieu de culture approprié, caractérisé en ce qu'il comprend une étape d'induction de la différenciation des cellules souches par inhibition de l'expression ou de l'activité du gène IPO6 exprimé dans lesdites cellules souches.

L'avantage de ce procédé est que les cellules différenciées obtenues sont homogènes. Les clones obtenus ne présentent plus la morphologie caractéristique d'un clone de cellules souches, mais une morphologie différenciée. Ces clones ne présentent plus non plus la prolifération caractéristique des cellules parentales et la cellularité reste constante après l'induction.

Cette induction de la différenciation des cellules souches est à la fois rapide et irréversible.

De façon préférée, les cellules aviaires sont issues d'un oiseau appartenant à l'ordre des Galliformes, en particulier d'un poulet ou d'une caille, et plus préférentiellement de l'espèce *Gallus gallus.*

Par « cellules souches », l'homme du métier comprend que les cellules présentent les caractéristiques suivantes :
- la capacité de proliférer en auto-renouvellement *in vitro* en présence de facteurs de croissance bien connus dans la littérature, et pendant des temps importants ;
- d'un point de vue morphologique, une cellule souche est caractérisée par un rapport nucléocytoplasmique fort, une taille relative de 10 à 15 µm avec un noyau de 5 à 10 µm, présente différentes activités biochimiques intrinsèques comme celles de l'alcaline phosphatase et de la télomérase, et est reconnue par différents anticorps spécifiques tels que décrits dans les brevets FR 94/12598 et FR 00/06029. Une autre particularité des cellules souches embryonnaires aviaires est leurs contacts membranaires étroits quand elles prolifèrent ;
- les cellules sont pluripotentes ce qui signifie qu'elles peuvent donner naissance à plusieurs types cellulaires, en particulier les cellules souches aviaires de l'invention peuvent se différencier en cellules de dérivés ectodermiques, mésodermiques et endodermiques.

Par « cellules différenciées », l'homme du métier comprend que les cellules prolifèrent moins ou plus du tout, qu'elles présentent un rapport nucléocytoplasmique plus faible, une taille en général plus grande (> 15- 20 µm ou davantage pour certains types cellulaires particuliers comme les ostéoclastes, les adipocytes matures, les cellules musculaires différenciées, certains macrophages, etc), avec une morphologie moins compacte et qu'elles ont perdu les marqueurs biochimiques des cellules souches non induites.

En particulier les cellules souches peuvent être différenciées en cellules épithéliales, caractérisées par la présence de marqueurs comme les cytokératines 8, 18 et 19 ou la kératine 16 pour les keratinocyte, certaines mucines, cadhérines et intégrines spécifiques. Selon l'origine des cellules épithéliales considérées, différentes associations de marqueurs seront caractéristiques de l'origine et du degré de différenciation des cellules épithéliales, notamment dans les processus néoplasiques (Barak et al., 2004).

Selon un autre aspect de l'invention, les cellules souches peuvent être différenciées en cellules hématopoiétiques précurseurs et différenciées, caractérisées entre autre par la présence de marqueurs membranaires, connus chez la souris et l'homme par la nomenclature CD (Cluster of Differentiation) et illustrée par Lai et al. dans une revue publiée dans le Journal of Immunology (1998). D'autres marqueurs non membranaires peuvent être également utilisés, notamment des facteurs de transcription spécifiques.

Par « inhibition de l'expression ou de l'activité d'un gène », l'homme du métier comprend que tous les moyens usuels permettant de contrôler l'expression des gènes sont utilisables, notamment l'utilisation d'inhibiteurs de transcription ou de traduction. En particulier l'usage d'ARN interférents permet d'inhiber spécifiquement l'expression de gènes d'intérêt.

Selon un aspect particulier de l'invention, l'inhibition de l'expression ou de l'activité du gène est réalisée de façon conditionnelle. Ainsi la différenciation des cellules peut être déclenchée à un moment souhaité par l'homme du métier.

Selon un aspect particulier de l'invention, l'inhibition de l'expression ou de l'activité du gène est réalisée au moyen d'au moins un ARN interférent.

Les molécules d'ARN interférent sont utilisées pour cibler spécifiquement des ARN messager (ARNm) : l'ARN interférent vient s'hybrider à l'ARNm et par conséquent inhibe la traduction de la protéine correspondante, soit par simple encombrement stérique, soit en favorisant le clivage de l'ARNm.

Cette stratégie d'inhibition de l'expression de gènes a été appliquée aux cellules embryonnaires souches murines à la fois *in vitro* et *in vivo* (Grabarek et al., 2003 ; Kunath et al., 2003 ; Lickert et al., 2005). L'utilisation des RNAi *in ovo* chez le poulet est possible comme cela a été démontré dans (Nakamura et al., 2004).

L'ARN interférent peut être choisi parmi plusieurs formes telles que : ARN antisens, ARN double-brin, « small interfering » ARN, « Small Hairpin» ARN ou ARN ribosomal.

Les siRNA, pour « Small Interfering RNA » ou ARN interférant de petite taille, sont des séquences courtes d'environ 15 à 30 paires de bases (bp), de préférence de 19 à 25 bp. Elles comprennent un premier brin et un brin complémentaire identiques à la région ciblée de l'ARN du gène cible. Par siRNA, on entend les formes d'ARN double brin synthétiques.

Les shRNA pour « Small Hairpin RNA » sont des ARN double brin produits par une séquence clonée dans un vecteur d'expression, codant pour une molécule d'ARN qui adoptera une forme en épingle à cheveux après clivage par le complexe Dicer et loquacious (Du et al ; 2005).

Le design et la préparation d'ARN interférents et leur utilisation pour la transfection de cellules in vivo et in vitro sont bien connues et largement décrites dans de nombreuses publications, telles que: US 6 506 559, US 2003/0056235, WO 99/32619, WO 01/75164, WO 02/44321, US 2002/0086356, WO 00/44895, WO 02/055692, WO 02/055693, WO 03/033700, WO 03/035082, WO 03/035083, WO 03/035868, WO 03/035869, WO 03/035870, WO 03/035876, WO 01/68836, US 2002/0162126, WO 03/020931, WO 03/008573, WO 01/70949, WO 99/49029, US 6573099, WO 2005/00320, WO 2004/035615, WO 2004/019973, WO 2004/015107, http://www.alugen.com/sirnatechnology.htm, http://www.alnylam.com/science-technology/index.asp, http://www.protocol-online.org./prot/Research Tools/Online Tools/SiRNA Design/, http://www.hgmp.mrc.ac.uk/Software/EMBOSS/Apps/sirna.html http://www.rockefeller.edu/labheads/tuschl/sima.html, http://www.upstate.com/browse/categories/siRNA.q.

Les siRNA peuvent être conçus et préparés en employant des logiciels appropriés disponibles en ligne, par exemple :
- "siSearch Program" http://sonnhammer.cgb.ki.se/siSearch/siSearch 1.6.html ("Improved and automated prediction of effective siRNA", Chalk AM, Wahlesdelt C, and Sonnhammer ELL, Biochemical and Biophysical Research Communications, 2004).
- "SiDirect" http://design.mai.jp/sidirect/index.php (Direct: highly effective, target-specific siRNA design software for mammalian RNA interference, Yuki Naito et al, Nucleic Acids Res, Vol. 32, No. Web Server issue © Oxford University Press 2004).
- "siRNA design tool" de Whitehead Institute of Biomedical Research au MIT http://jura.wi.mit.edu/pubint/lhttp://iona.wi.mit.edu/siRNA-ext/
- siRNA wizard^{™} de Invitrogen http://www.sirnawizard.com/.
- "siRNA Target Finder" de Ambion http://www.ambion.com/techib/misc/siRNA finder.html,
- https://www.genscript.com/ssl-bin/app/mai
- http://www.promega.com/siRnADesigner/default.htm
- http://bioweb.pasteur.fr/seganal/interfaces/sima.html
- D'autres programmes sont référencés sur le site http://web.mit.edu/mmcmanus/ivww/homel.2files/siRNAs.htm, comme http://athena,bioc.uvic.ca/cgi-bin/emboss.pl? action=input& app=sirna.

Les outils pour la préparation de siRNA et la transfection de cellules sont à la disposition du public, comme par exemple les vecteurs de siRNA commercialisés par la société Invitrogen (httn://www.invitrogen.com).

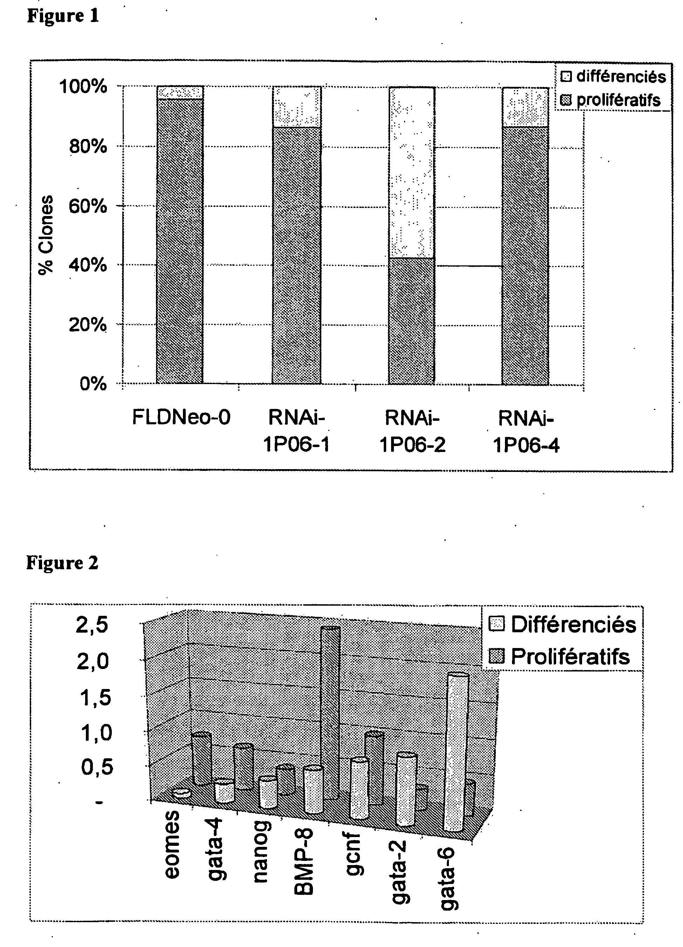

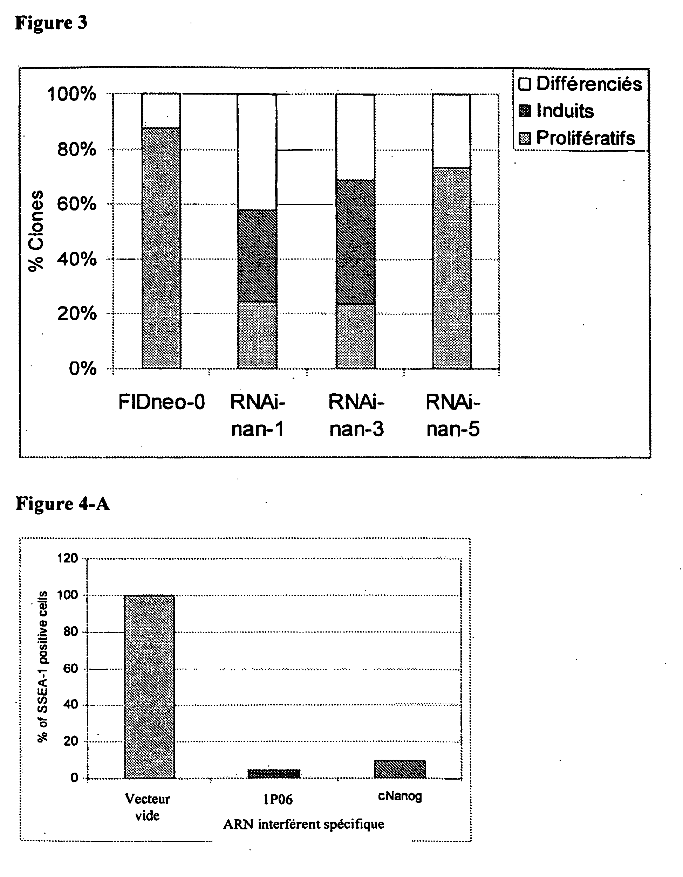

Selon les séquences d'ARN interférents sélectionnées par l'homme du métier, différents niveaux d'inhibition pourront être obtenus, permettant de moduler l'effet inhibiteur recherché. De manière préférentielle, les ARN interférents sont préparés et sélectionnés pour obtenir au moins 50 % d'inhibition de l'expression du gène cible dans une cellule, voire au moins 75%, 90%, 95% voire plus de 99% d'inhibition. L'homme du métier saura sélectionner les ARNi les plus efficaces par simple travail de routine, par exemple en analysant leur capacité à induire la différenciation des cellules souches (voir Figures 1 et 3).

l'ARN interférent inhibe spécifiquement l'expression du gène 1P06.

En particulier, la molécule d'acide nucléique codant pour l'ARN interférent peut présenter une séquence telle que présentée dans la liste de séquences sous l'intitulé SEQ ID N° 3.

Une technique courante pour inhiber l'expression d'un gène avec un ARN interférent consiste à introduire dans la cellule un ARN interférent double brin ; mais, comme cet ARNdb sera dégradé rapidement, l'inhibition du gène sera limitée dans le temps. Cette limitation a conduit les inventeurs à rechercher un système permettant de produire dans la cellule cible des ARN interférents « à la demande ».

La présente invention concerne des vecteurs d'expression comprenant une molécule d'acide nucléique codant pour un ARN interférent, placée sous le contrôle d'un promoteur permettant l'expression dudit ARN interférent dans une cellule hôte. La cellule hôte sera préférentiellement une cellule souche aviaire maintenue en culture *in vitro.*

Lesdits vecteurs comprennent de préférence un promoteur, des signaux d'initiation et de terminaison de la traduction, ainsi que des régions appropriées de régulation de la transcription.

Selon un mode particulier de l'invention, le promoteur du vecteur d'expression qui contrôle l'expression de l'ARN interférent est un promoteur inductible.

L'avantage d'un système d'expression inductible est que l'on peut maîtriser la durée de présence de l'ARN interférent, et donc de l'inhibition du gène cible. En effet, certains gènes, essentiels à la survie de la cellule, ne peuvent être inhibés trop longtemps car cela induirait la mort cellulaire ; d'autres gènes doivent être inhibés pendant une longue période avant de pouvoir observer les effets de cette inhibition. La maîtrise de l'expression de l'ARN interférent au sein de la cellule hôte permet de contourner ces deux contraintes.

L'homme du métier connaît plusieurs types de promoteurs transcriptionnels inductibles, comme par exemple celui de l'opéron lactose, qui a été le premier identifié et analysé. On peut également citer le système tet off/tet on basé sur un promoteur dérivant de l'opéron de résistance à la tétracycline de E.coli (Gossen et al., 1992).

L'induction de l'activité d'un promoteur peut être réalisée soit en activant un promoteur inactif, soit en levant l'inhibition pesant sur un promoteur actif constitutionnellement. Chaque facteur inhibiteur ou activateur de transcription peut lui-même être inductible en fonction de l'environnement cellulaire.

Par exemple, si le promoteur possède un domaine de fixation à des facteurs inhibiteurs de la transcription, la levée de l'inhibition se fera par le piégeage de ces facteurs qui ne pourront plus se fixer sur les séquences régulatrices du promoteur ; le promoteur sera alors « libéré » des facteurs inhibant la transcription.

Selon un autre exemple, le promoteur est actif constitutionnellement, mais est inhibé par la présence d'une cassette insérée dans son locus ; la cassette est placée de telle façon que les éléments régulateurs du promoteur ne peuvent plus coopérer entre eux car ils sont trop éloignés ; la levée de l'inhibition se fait alors en excisant la cassette, en particulier au moyen de séquences reconnues par une recombinase, placées à chaque extrémité de la cassette. La cassette peut comprendre une longue séquence d'ADN « tampon » servant uniquement à faire de l'encombrement stérique (Tiscomia et al., 2004), mais elle peut aussi comprendre des gènes « marqueurs » comme par exemple des gènes de résistance aux antibiotiques, ou des gènes de fluorescence comme la GFP (Green Fluorescence Protein). La présence de cette cassette de sélection permet également de maintenir le locus dans une configuration chromatinienne active qui limite les mécanismes de 'silencing' des séquences, notamment dans les cellules souches.

Pour obtenir l'excision de la cassette, deux systèmes sont fréquemment utilisés, le système Cre-Lox et le système FLP-FRT. Le système Cre-lox est basé sur l'habilité d'une enzyme, la Cre recombinase, de supprimer tout fragment d'ADN se trouvant entre des séquences précises d'ADN : les séquences loxP. Le système FLP-FRT est lui basé sur l'activité de la FLP recombinase qui supprime les fragments d'ADN se trouvant entre deux séquences FRT. Les sites de reconnaissance Lox P et FRT et les recombinases Cre et FLP correspondantes sont bien connus de l'homme du métier, notamment décrit dans Kilby, et al. ; 1993, Sauer et Henderson, 1988, Gu et al. ; 1994, Cohen-Tannoudji et Babinet ; 1998, Shibata et al. ; 1997, Schlake et Bode 1994.

En particulier, un promoteur inductible peut contenir une cassette lox - promoteur de la Thymidine kinase (TK)-Neomycin^{R}-lox dont la présence inhibe son fonctionnement (Coumoul et al., 2004). Le promoteur ne devient fonctionnel qu'après excision par la recombinase CRE de cette cassette. La recombinase utilisée CRE a elle-même une activité dépendante de la présence de tamoxifène dans le milieu de culture (Metzger et al., 1999). Ainsi par ajout de tamoxifène dans le milieu de culture des cellules, la recombinase devient active et excise la séquence inhibitrice du promoteur, rendant ainsi le promoteur fonctionnel, permettant ainsi l'expression de l'ARN interférent.

Parmi les promoteurs d'intérêt, on choisit de préférence les promoteurs pol III dépendants comme le promoteur U6 ou H1, bien connus de l'homme de métier pour leur utilisation dans l'expression des ARN interférents.

Le vecteur d'expression selon l'invention peut être intégré dans une cellule hôte, en particulier une cellule aviaire maintenue en culture *in vitro.* On dit alors que les cellules sont « transformées ». Pour se faire, on peut utiliser des vecteurs à réplication autonome au sein de la cellule hôte, ou des vecteurs intégratifs qui vont permettre l'intégration de la molécule d'acide nucléique exogène dans l'ADN de la cellule hôte.

Parmi les systèmes à réplication autonome, on utilise de préférence des systèmes de type plasmidique ou viral, les vecteurs viraux pouvant notamment être des adénovirus (Perricaudet et al., 1992), des rétrovirus, des lentivirus, des poxvirus ou des virus herpétiques (Epstein et al., 1992). L'homme du métier connaît les technologies utilisables pour chacun de ces systèmes.

Lorsque l'on souhaite l'intégration de la séquence dans les chromosomes de la cellule hôte, on peut utiliser par exemple des systèmes de type plasmidique ou viral; de tels virus sont, par exemple, les rétrovirus (Temin, 1986), ou les AAV (Carter, 1993).

Parmi les vecteurs non viraux, on préfère les polynucléotides nus tels que l'ADN nu ou l'ARN nu selon la technique développée par la société VICAL.

Dans les cellules aviaires, on utilisera préférentiellement des rétrovirus, des adénovirus aviaires, des poxvirus ou bien de l'ADN nu introduit par transfection ou électroporation.

De tels vecteurs sont préparés selon les méthodes couramment utilisées par l'homme du métier, et les clones en résultant peuvent être introduits dans une cellule hôte par des méthodes standard, telles que la lipofection, l'électroporation, le choc thermique, la transformation après perméabilisation chimique de la membrane ou la fusion cellulaire.

Selon un autre aspect de l'invention, le procédé est caractérisé par le fait que les cellules subissent au moins une étape d'activation ou d'inhibition de gènes spécifiques, dans le but d'obtenir une différenciation spécifique desdites cellules.

En particulier, l'utilisation de certains promoteurs et phases codantes permet de contrôler l'expression de gènes qui induisent eux-mêmes la différenciation terminale des cellules dans des voies définies. Ces gènes codent en particulier pour des facteurs de transcription. Parmi ces gènes, on peut citer les régulateurs du lignage myogénique ('MRF') comme *Myf5, MyoD, Mrf4,* ceux impliqués dans le déterminisme de la lignée adipocytaire comme le gène PPARg, dans le déterminisme neuronal comme *Sox1* et *Nestine,* dans le déterminime de la voie hématopoiétique comme *Gata2* et *Gata3.*

Cette étape d'activation ou d'inhibition de l'expression ou de l'activité de gènes peut être soit simultanée à l'étape d'inhibition du gène *1P06,* soit décalée dans le temps, et de préférence suivre de quelques heures l'induction de l'inhibition. Ce décalage permet à la cellule de mettre en route le programme de différenciation terminale de façon séquentielle et ainsi de mieux reproduire les cinétiques de différenciation observées *in vivo*.

Selon un autre aspect du procédé de l'invention, les cellules souches aviaires de l'invention sont cultivées dans des conditions appropriées, en particulier en présence de certains facteurs de croissance et inducteurs, pour obtenir une différenciation spécifique desdites cellules.

Parmi ces facteurs, on peut citer les facteurs de croissance de la famille des BMP, des FGF et de la famille wnt dont les rôles relatifs très complexes dans le contrôle des voies mésodermiques et ectodermiques commencent à être décrits à la fois chez la souris, le xénope et le poulet. On utilisera de préférence ces facteurs dans une gamme de concentration de l'ordre de 1 à 10 ng /ml, notamment pour le BMP-4 et le BMP-7. De très nombreux autres facteurs interviennent dans les inductions des voies ecto et mésodermiques.

Le procédé selon l'invention peut également comprendre une étape de sélection et destruction des cellules non-induites en différentiation.

La disparition des clones non induits peut être réalisée de façon concomitante à l'induction, par une sélection négative à l'aide d'un vecteur exprimant un gène toxique (thymidine kinase, toxine diphtérique), placé sous contrôle d'un promoteur actif uniquement dans les cellules souches, par exemple le promoteur du gène *Ens1* ou du gène *1P06* décrit ci-après. Ainsi seules les cellules présentant les caractéristiques de cellules souches disparaîtront.

24 à 72h après l'induction de la différenciation des cellules souches, les cellules sont mises en présence avec le virus d'intérêt dans un milieu de culture adéquat. Il est nécessaires de mettre le virus en contact avec les cellules pendant que celles-ci sont dans leur phase d'induction pour présenter une division résiduelle permettant l'intégration du virus et sa production, même si les cellules induites ne se divisent que faiblement par rapport aux cellules en prolifération. Le temps de doublement passe de 12 à 15 heures environ à plus de 24 à 36 h dans la phase précoce d'induction pour aboutir à une cellule qui ne se divise plus au-delà de 96 h après le début de l'induction. Les m.o.i nécessaires à l'infection sont calculées sur le nombre de cellules obtenu après 24 à 48 h après le début de l'induction.

Les cellules souches et leurs dérivées différenciées peuvent servir de support pour la réplication de différents virus en fonction du tropisme particulier de ces derniers. Cette stratégie peut être développée avec succès avec les cellules souches au regard de leur grande plasticité phénotypique. L'adaptation de beaucoup de virus à des substrats cellulaires uniformes de laboratoire (comme les cellules des lignées BHK-21, CV-1 par exemple) modifie les rendements de production des virus en affectant leur cycle réplicatif. Les mécanismes contrôlant ces restrictions commencent à être élucidés (Wang & Shenk, 2005).

Un autre objet de l'invention est une molécule d'acide nucléique codant pour un ARN interférent tel que précédemment défini dans l'invention.

L'invention concerne également un vecteur d'expression comprenant une molécule d'acide nucléique codant pour un ARN interférent selon l'invention, placée sous le contrôle d'un promoteur permettant l'expression dudit ARN interférent dans une cellule hôte, de préférence une cellule aviaire maintenue en culture *in vitro.*

Selon un aspect préféré de l'invention, ce promoteur est inductible.

L'invention se rapporte également aux cellules souches aviaires transformées avec un vecteur d'expression tel que décrit ci-dessus.

Les exemples ci-dessous permettent d'illustrer l'invention, et ne doivent pas être considérés comme la limitant.

### DESCRIPTION DES FIGURES

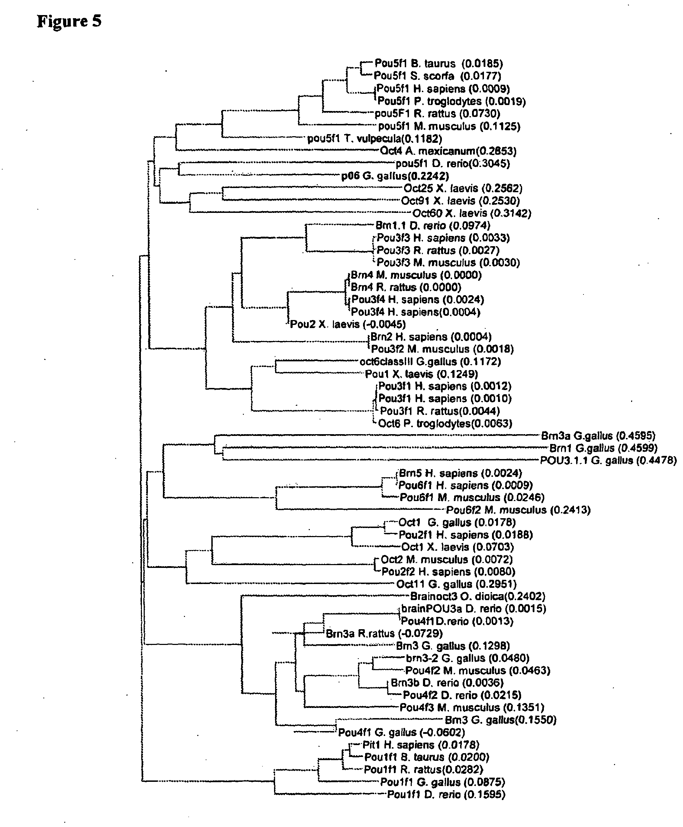

**Figure 1**: L'inhibition de l'expression du gène *1P06* par des RNAi spécifiques induit une perte drastique de la prolifération des cellules CESC et induit leur différenciation. Cet effet est mesuré sur la morphologie des clones dont la différenciation a été induite par l'hydroxy-tamoxifene.
**Figure 2** : Analyse moléculaire des clones différenciés obtenus par l'inhibition de l'expression du gène *1P06* par le RNAi-1P06-2. Le contenu transcriptomique des clones différenciés ou prolifératifs est analysé par PCR en temps réel.
**Figure 3** : L'inhibition de l'expression du gène *Nanog* par des RNAi spécifiques induit la différenciation des cellules CESC et donc un arrêt de la prolifération cellulaire. Cet effet est mesuré sur la morphologie des clones dont la différenciation a été induite par l'hydroxy-tamoxifene.
**Figure 4** : Inhibition de l'expression des gènes *1P06* et *Nanog* par un ARN interférent spécifique :
   A- Marquage des cellules induites en différenciation par l'anticorps anti-SSEA-1
   B- Cinétique de l'arret de la prolifération induit par les ARN interférents
   C- Analyse moléculiare de l'expression de gènes marqueurs de différenciation dans les cellules après l'induction de cette différenciation
**Figure 5** : Arbre de phylogénie des différents membres de la multifamille POU, comprenant les orthologues et paralogues du gène *1P06* de G. gallus.

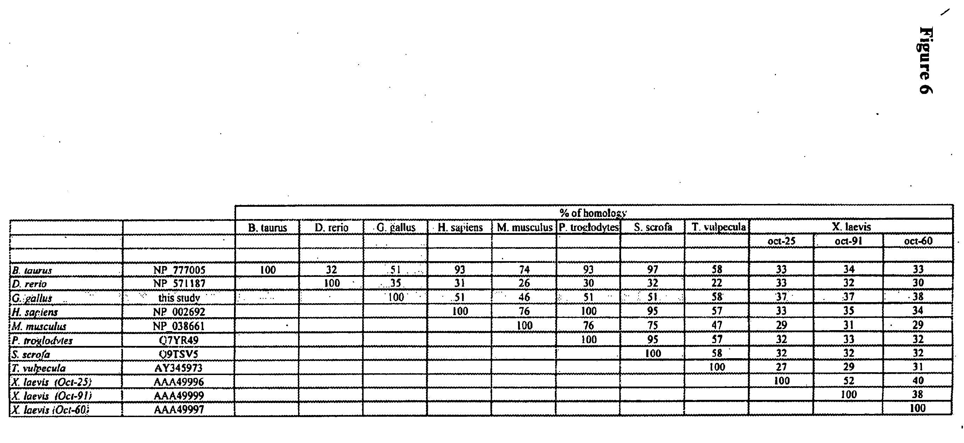

**Figure 6** : Pourcentages d'homologie du gène *1P06* de G. gallus avec ses orthologues de différentes espèces.

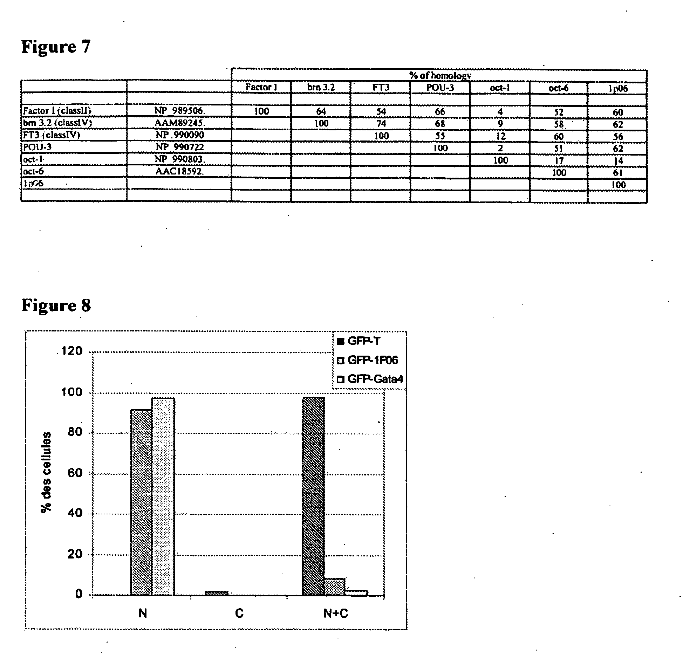

**Figure 7** : Pourcentages d'homologie du gène *1P06* avec d'autres facteurs de la famille POU dans l'espèce G. gallus.
**Figure 8** : Localisation de la protéine de fusion GFP-1P06 dans des cellules CESC en prolifération (N : nucléaire ; C : cytoplasmique ; N+C : nucléaire et cytoplasmique).

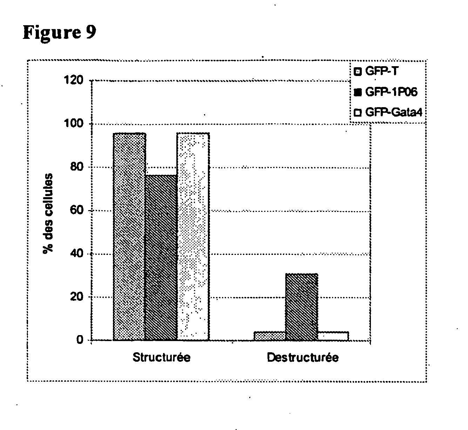

**Figure 9** : Impact de la sur-expression de la protéine de fusion GFP-1P06 dans des cellules CESC en prolifération. Le niveau de structuration de la chromatine est déterminé.

### EXEMPLES

### EXEMPLE 1 : INDUCTION DE LA DIFFERENTIATION DES CELLULES SOUCHES AVIAIRES PAR INHIBITION DU GENE 1P06 AU MOYEN D'UN ARN INTERFERENT

### a) Construction du vecteur d'expression codant pour l'ARN interférent

Le plasmide pFLΔNeo est obtenu par insertion du fragment mU6ΔneoD de 2kb, amplifié par PCR dans les sites SmaI/HindIII du pBSK (Stratagene). Ce fragment mU6ΔneoD est amplifié avec les oligonucléotides primers m-U6-smaI-S et m-U6-HindIII-AS à partir de la matrice mU6D-Neo-DApaIDXhoI qui nous a été fourni par le Dr. Sheng, NIH Bethesda et qui est décrite dans l'article (Coumoul et al., 2004).

Une fois dessinés à l'aide des logiciels fournis sur les sites http://wwwproligo.com; http://www.qiagen.com, http://www.ambion.com, 100pM de chaque oligonucléotide RNAi sont hybridés pour obtenir le fragment double brin par renaturation lente à température ambiante en présence de 1mM MgCl₂ après une dénaturation par chauffage à 95°C pendant 5 min. L'oligonucléotide double brin résultant est cloné directement dans les sites HindIII/XhoI du vecteur pFLΔNeo préparé par digestion avec ces mêmes enzymes. Le criblage est validé par la disparition du site HincII et par un séquençage systématique de ces plasmides positifs. Pour chaque RNA 'X' cible, on obtient ainsi le vecteur pFLΔNeo X-RNAi. La séquence des différents RNAi utilisés est présentée dans la liste de séquences. Avant transfection, les vecteurs sont linéarisés par AhdI, site unique présent dans le gène de résistance à l'ampicilline.

Le vecteur d'expression de la recombinase CRE-ERT2-hygro dérive du vecteur commercial pCI-Neo (Promega) à la suite de plusieurs étapes. La cassette de résistance à l'hygromycine est obtenue à partir du vecteur commercial pIRES-Hygro (Clontech) par amplification PCR à l'aide de la paire d'oligonucléotides NruI-hygroS et BamHI-hygro AS. Le fragment résultant est directement cloné en NruI-BamHI dans le vecteur pCl-Neo dans lequel la cassette de résistance à la néomycine a été au préalable enlevée par une digestion KpnI -BamHI. Le plasmide résultant est le plasmide pCI FLHygro. La phase codante de la recombinase CRE-ERT2 est amplifiée à partir du plasmide pCRE-ERT2 fourni par le Pr. P. Chambon, IGMC, Strasbourg et décrit dans la publication Feil and al., 1997 en utilisant la paire d'oligonucléotide CRE-ERT2-SalI S et CRE-ERT2-smal AS. Le fragment amplifié résultant est directement cloné dans le vecteur receveur pCI FL-Hygro préparé par une digestion par SalI-SmaI. Le clonage est directionnel et il en résulte le vecteur pGRE-ERT2-Hygro. L'ensemble des phases codantes est séquencé afin de vérifier l'intégralité des amplifications et permettre une bonne activité de la recombinase.

### b) Introduction du vecteur d'expression comprenant l'ARN interférent dans les cellules souches aviaires

Les cellules CESC sont obtenues, amplifiées et maintenues in vitro comme décrit précédemment dans Pain et al., 1996 et les demandes FR N°94/12598, FR N°00/06029. Pour les transfections, 0,5 à 1 x 10⁶ cellules sont transfectées à l'aide de liposomes (Fugène 6, Roche) comme décrit précédemment (Pain et al., 1999, Demandes de brevet FR N° 00/06029 et FR N° 01/15111) à l'aide de 2 à 10 µg des différents vecteurs linéarisés. La première étape consiste à transfecter les vecteurs d'expression pFLΔNeo X-RNAi. La sélection à la néomycine (de 100 à 250 µg/ml) est appliquée pendant 7 jours. Les clones résistants obtenus sont comptés et peuvent être prélevés, amplifiés et congelés individuellement pour permettre une utilisation ultérieure après amplification. Ils ont intégré le vecteur d'expression du RNAi, mais ne l'expriment pas. Ces clones sont ensuite dissociés et soumis à une nouvelle transfection par le vecteur d'expression de la recombinase pCRE-ERT2-Hygro. Les cellules sont sélectionnées en présence d'hygromycine (de 25 à 75 µg/ml) avec une présence maintenue d'une sélection à la néomycine (de 50 à 200µg/ml). Après 7 jours, les clones obtenus sont à nouveau comptés, prélevés et amplifiés individuellement.

### c) Expression conditionnelle de l'ARN interférent dans les cellules souches

Le vecteur pFLD Neo XRNAi permet de contrôler l'expression des RNAi par la présence d'une séquence lox-TK-Neo-lox, dans le promoteur U6 murin, séquence qui rend conditionnel le fonctionnement de ce promoteur (Coumoul et al., 2004). Le promoteur devient fonctionnel après excision par la recombinase CRE de cette insertion. Nous avons utilisé en particulier une recombinase CRE-ERT2 dont l'activité est dépendante de la présence de tamoxifène dans le milieu de culture (Metzger et al., 1999).
Sur les clones obtenus après les deux étapes de transfections décrites, l'addition de 1 µM de 4-hydroxy-tamoxifene dans le milieu de culture pendant 96h induit l'excision de la cassette de résistance à la néomycine, encadrée par les sites loxP, et permet la production du RNAi cible.

### d) Caractérisation morphologique des cellules différenciées obtenues

Après induction par le 4-hydroxy-tamoxifene de l'expression de la recombinase CRE, la morphologie des cellules obtenues est analysée par observation microscopique directe et enregistrement à l'aide d'une Caméra CCD CoolSNAP (Photometrix).
Les clones sont comptés 4 jours (96 heures) après l'induction par le 4-hydroxy-tamoxifene ; les résultats obtenus sont représentés sur la figure 1. Le nombre total de clones obtenus dans 3 expériences indépendantes est respectivement de n = 45 pour le pFLΔNeo-0 (vecteur contrôle vide), n = 59 pour pFLΔNeo-1, n = 61 pour pFLΔNeo-2 et n = 76 pour pFLΔNeo-4.
La figure 1 montre le pourcentage de clones dont la différenciation a été induite et le pourcentage de clones qui sont toujours en prolifération. Seul le RNAi-2 permet l'obtention du phénomène d'induction à l'inverse du vecteur contrôle vide et des RNAi-1 et -4, pour lesquels aucun changement phénotypique majeur n'est observé, comme le montre le nombre de clones non différenciés.
Ainsi, même en présence des facteurs de croissance et cytokines nécessaires à la prolifération des cellules non différenciées, on observe dans les cellules exprimant le RNAi-2 un changement rapide et drastique de la morphologie des cellules dans les clones. La capacité prolifératrice de ces clones est perdue et ils deviennent proportionnellement plus petits par rapport aux clones non induits. Ces clones non induits peuvent résulter d'une non excision de la cassette lox par la recombinase, et donc d'une non expression du RNAi.
Les cellules induites présentent une morphologie plate, avec une plus grande taille que les cellules parentales non induites. Des observations en microscopie en balayage montrent également un plus grand étalement des cellules induites.

### e) Caractérisation moléculaire des cellules induites

Afin de caractériser au niveau moléculaire les clones induits par l'expression du RNAi inhibant l'expression du gène *1P06,* des clones différenciés et non différenciés ont été prélevés et analysés. Les résultats obtenus sont présentés figure 2. Le niveau d'expression de différents gènes marqueurs est représenté. Le niveau 1 est celui observé dans les clones ayant intégré le vecteur vide pFLDNeo-0.

L'analyse du contenu transcriptomique de ces clones par des approches de RT-PCR en temps réel indique que le niveau global d'expression du gène *1P06* est moins important dans ces clones par rapport aux cellules parentales (résultat non montré). Les niveaux d'expression des gènes *Nanog, Eomes* et *Gcnf* sont diminués. A l'inverse, le gène *Gata6* voit son expression augmentée, suggérant une induction dans une voie de différenciation équivalente à l'endoderme primitif.

### EXEMPLE DE REFERENCE 2: INDUCTION DE LA DIFFERENCIATION DES CELLULES SOUCHES AVIAIRES PAR INHIBITION DE L'EXPRESSION DU GENE NANOG AU MOYEN D'UN ARN INTERFERENT

En réalisant des expériences similaires que celles réalisées sur le gène *1P06,* avec des vecteurs d'expression de RNAi dirigés contre le gène *Nanog,* on observe également une inhibition de la prolifération et une induction de la différenciation des clones sélectionnés. Les clones sont comptés 4 jours (96 heures) après l'induction de la recombinase CRE par l'addition du 4-hydroxytamoxifene. Le nombre total de clones obtenus est respectivement de n = 5 pour le pFLΔNeo-0 (vecteur contrôle vide), n = 21 pour pFLΔNeo-nan-1, n = 37 pour pFLΔNeo-nan-3, n = 15 pour pFLΔNeo-nan-5. Les résultats obtenus sont montrés sur la figure 3.
La figure 3 montre le pourcentage de clones dont la différenciation a été induite (clones induits ou différenciés) et le pourcentage de clones qui sont toujours en prolifération. A l'inverse du vecteur vide, les RNAi-nan1 et RNAi-nan3 sont capables d'induire une différenciation de la majorité des cellules : moins de 30% des clones sont toujours en prolifération. Le RNAi-nan-5 induit également cette différenciation, dans une moindre mesure.

### EXEMPLE 3 : INDUCTION DE LA DIFFERENCIATION PAR INHIBITION DES GENES 1P06 ET NANOG EN PARALLELE

Dans ces expériences, les shRNA utilisés ont été les suivants :
RNAi-2 pour l'inactivation de *1P06,* et RNAi-nan1 pour l'inactivation de *Nanog*

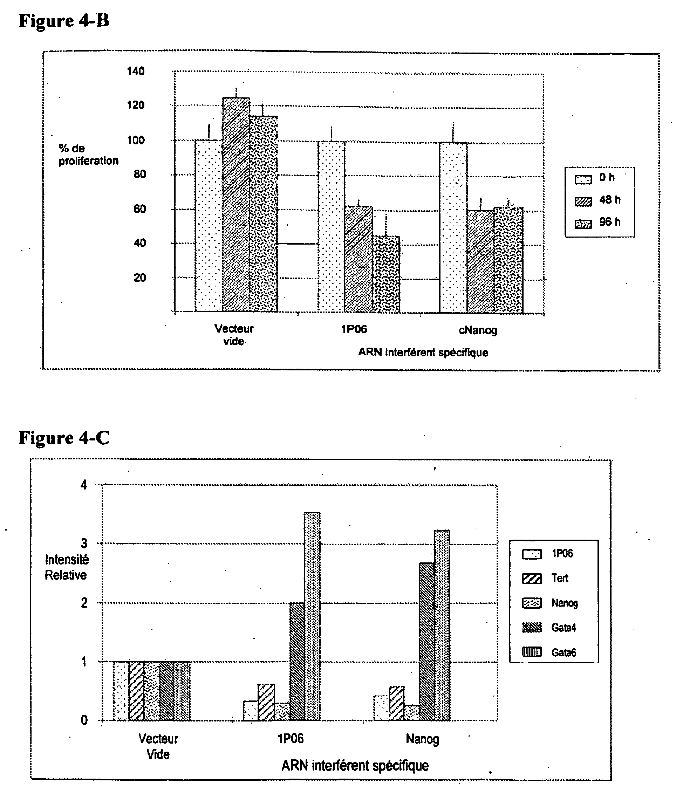

La différenciation des cellules souches aviaires, suite à l'induction par inhibition de l'expression des gènes *1P06* et *Nanog,* a été évaluée par les mesures suivantes :
- pourcentage de cellules positives au marquage par l'anticorps anti-SSEA-1 (Figure 4A)
- pourcentage de prolifération des clones obtenus sur 48 et 96 heures (Figure 4B)
- expression relative de gènes «marqueurs » : *1P06, Tert, Nanog, Gata4, Gata6* (Figure 4C).

La figure 4A montre que les cellules obtenues, en grande majorité, n'expriment plus l'antigène SSEA-1 (Stage-Specific Embryonic Antigen-1), dont l'expression est caractéristique des cellules souches (100% de cellules positives lorsque la transfection est réalisée avec un vecteur vide).
La figure 4B met en evidence la chronologie des événements, et notamment le fait que l'arrêt de la prolifération de clones est obtenu rapidement.
Les clones sont comptés par observation microscopique directe, comme décrit précédemment, à 48 et 96 heures après l'induction par le 4-hydroxy-tamoxifene ; la figure y montre le pourcentage relatif de clones dont la différenciation a été induite ou qui sont toujours en prolifération : après 48h seulement 60% des clones sont encore en prolifération, ceci étant l'effet maximal obtenu en inhibant l'expression du gène *Nanog ;* après 96h d'inhibition du gène *1P06,* on n'observe plus que 40% de clones en prolifération.

La figure 4C montre la caractérisation moléculaire des cellules induites en différenciation par inhibition de l'expression des gènes *1P06* et *Nanog.*
L'expression des différents gènes « marqueurs » est normalisée par rapport au taux d'expression '1' observé dans les clones ayant intégré le vecteur vide pFLDNeo-0.
L'analyse du contenu transcriptomique de ces clones par des approches de RT-PCR en temps réel indique que le niveau global d'expression du gène *1P06* est moins important après différenciation. Les niveaux d'expression des gènes *Tert* et *Nanog* sont diminués. A l'inverse, les gènes *Gata-4* et *Gata-6* ont une expression augmentée, suggérant une induction dans une voie de différenciation équivalente à l'endoderme primitif.

### EXEMPLE 4 : CLONAGE DU GENE 1P06

### Oligonucléotides et séquençage

Tous les oligonucléotides ont été dessinés à l'aide du logiciel Primer 3 (http://frodo.wi.mit.edu/cgi-bin/primer3/primer3 www.cgi, puis synthétisés par la société Proligo. Les séquences des différents gènes testés sont identifiés dans l'index TIGR (littp://www.tigr.orp-/tigr-scripts/tgi/T index.cgi?species=g gallus) ou recherchées directement sur le site d'assemblage du génome du poulet (http://www.ensembl.orp,/Gallus gallus/) ou otenus directement à partir des séquences nouvellement identifiées.

### Extraction de RNA

Les RNAs totaux des cellules non différenciées en prolifération ou des cellules CESC induites en différenciation en corps embryoïdes pendant 2 jours sont extraits à l'aide du kit RNAEasy mini (Quiagen, réf 74104). Les RNA totaux des clones sont extraits à l'aide du kit RNA Easy micro (Qiagen ref 74004). La quantité et la qualité des RNA sont mesurées par le BioAnalyser 2100 (Agilent Biotechnologies).

### Hybridation liquide soustractive

Après une étape de transcription réverse, les cDNA sont soumis à la digestion par l'enzyme de restriction Sau3A et deux populations sont constituées selon le type d'amorce qui est ajouté par ligation. L'une est utilisée pour épuiser les messagers présents dans l'autre et vice versa. Ces cDNA sont alors utilisés comme matrice avec des oligonucléotides complémentaires de l'une ou de l'autre population et amplifiés dans des conditions qui préservent la représentativité de chaque échantillon. Après amplifications, les amplicons sont mélangés dans un ratio 1:4 pour l'une et l'autre population. Une des deux amorces contient un élément de biotinylation en 5'. Après le mélange, les DNA sont précipités dans l'éthanol, centrifugés et repris dans un petit volume réactionnel pour favoriser la renaturation qui a lieu pendant 20 heures après une courte étape de dénaturation. Des billes couplées à la streptavidine sont ajoutées pour éliminer les DNA 'drivers' biotinylés et les fragments monocaténaires du tester isolés par l'addition de la protéine SSB (Single Strand DNA binding protein). De l'ADN est à nouveau ajouté et le cycle d'enrichissement de l'ADN monobrin répété à nouveau deux fois. Sur l'ADN obtenu à l'issue de ces étapes, une réaction de PCR est réalisée avec des olgonucléotides spécifiques. Cette réaction permet l'amplification des cibles exprimées de façon différentielle. Les fragments obtenus sont soumis à une digestion par BamHI et sous clonés dans le plasmide pUC18. Après transformation et amplification, des colonies sont prélevées individuellement, l'ADN plasmidique extrait et soumis avec l'amorce universelle M13rev à une réaction de séquence avec le BigDye Teminator Cycle Sequencing kit sur AB1 Prism 3730 Sequencer.
L'ensemble de cette procédure de clonage et d'identification des inserts par comparaison avec les données de banques comme les données TIGR (http://wvww:tigr.org/tigr/T index.cgi?species=g_gallus) a été réalisé par la société Genome Express (http://www.genome-express.com/, Meylan).

### Criblage de banque et clonage du gène 1P06

Les RNA totaux des cellules CESC non différenciées en prolifération sont utilises pour construire une banque de cDNA dans le vecteur λZAP-II (Stratagene) ouvert en EcoRI et déphosphorylé par un traitement à la CIAP (Stratagene) et préalablement décrit dans la demande FR00/06009 et Acloque et al., 2001. 150 ng de l'ADN de phage est criblé par PCR génomique en utilisant 40 pM de différentes paires d'oligonucléotides associant soit l'oligonucléotide T7, soit T3 présent dans le vecteur λZap et les oligonucléotides pou(1P06)S ou pou(1P06)AS dérivés du clone 1P06G01. Les conditions d'amplification sont une dénaturation 5min à 96°C, puis 10 cycles composés de 30s à 95°C, 30s à 54°C et 1 min à 72°C suivis de 20 cycles de30s à 96°C, 30s à 54°C et 1 min à 72°C. La dernière étape d'élongation est maintenue pendant 7min. Le produit d'amplification est soumis à une électrophorèse sur gel d'agarose, purifié par le kit Gel Extraction Kit (Qiagen), sous cloné dans le vecteur pGEM-Teasy (Promega) et séquençé. Le clone pL9-4 contient un codon stop à 40 bp après la fin du clone IP06g01 initial et le clone pL7-2 permet l'obtention de 350 bp en amont de la séquence.

Une stratégie 5'RACE est développée à l'aide du kit 5'RACE (Invitrogen). 2µg de RNA totaux sont reverse transcrits pendant 1h à 42°C avec 10pM de l'amorce 1P06G01(RAAS2). Une fois le produit purifié, une queue de polyA est ajoutée par l'action de la Terminal transférase TdT pendant 30 min à 37°C (Invitrogen). 1/5 de la réaction est utilisé comme matrice dans une réaction de PCR en présence de 20pM des amorces 1P06(RAAS1) et AnchPrimSeq. Les paramètres de la réaction sont une dénaturation à 94°C pendant 2 min suivie de 30 cycles composés d'une dénaturation de30s à 94°C, d'une hybridation de 30s à 55°C et d'une phase d'élongation de 2 min à 72c à l'exception de la dernière réaction qui dure 7 min. Un second ensemble d'amplification est réalisé sur 5 µl du produit dilué 100 fois en utilisant les amorces P06 (pL7-2)AS et PCRprimseq. Le produit final est purifié après migration par électrophorèse puis sous cloné dans le vecteur pGEMTeasy. Le clone R1-8 résultant contient 300 pb en amont du clone pL7-2 avec un ATG en phase. Il en résulte un cadre de lecture ouvert de 888 bp.

### RT-PCR

2 µg de RNA totaux sont reverse transcripts pendant 1h à 42°C en utilisant une amorce oligodT et l'enzyme Superscript II (Invitrogen). La PCR en temps réel est réalisée sur le système MXP 3000P PCR (Stratagene). 2 µl du mélange dilué 100 x sont mélangés dans un volume final de 25 µl avec 12,5 µl du tampon Mix Quantitect SYBRGreen (Qiagen) en présence de 10 pM de chaque amorce (Proligo). Chaque échantillon est testé en triplicat dans une plaque de 96 puits (réf. 410088 Stratagene). Les paramètres sont : une étape de dénaturation de 15 min à 95°C, suivie de 40 cycles composés d'une dénaturation de 30s à 95°C, d'une renaturation de 30s à 55°C et d'une étape d'élongation de 30s à 72°C. Le niveau d'expression des gènes est calculé par la méthode du ΔΔCt selon une procédure détaillée disponible sur le site : http://www.gene-quantification.info. Le niveau d'expression du gène ribosomal RS17 (X07257) est utilisé comme référence interne entre les échantillons.
Le résultat des différentes étapes est l'identification d'un premier fragment de 228 bp qui contient un domaine POU puis d'une séquence de 888 bp (SEQ ID N°2) à l'issue des étapes de criblage de la banque et de la stratégie 5'RACE. Cette séquence code pour une protéine de 296 acides aminés (SEQ ID N°1). Le domaine POU de la protéine ainsi identifiée commence à l'acide aminé n° 90, et est composé de 149 acides aminés.
En comparant les séquences connues et identifiées dans les banques de données de gènes de différentes espèces qui comprennent un domaine POU, on peut représenter le positionnement phylogénétique de du nouveau gène identifié chez le poulet, ci-après dénommé *1P06 :* voir figures 5, 6 et 7.
Grâce au logiciel NTiVector (InVitrogen), en alignant les 888 paires de bases de la phase codante du gène *1P06* du poulet G. gallus avec les séquences des différents gènes *Pou5f1,* notamment les séquences des gènes de :
1155 bp de la séquence du tunicier O. dioica (AY613856), les 1134 bp de la séquence de la souris M. Musculus *Pou5f1* (NM 013633), les 1615 bp du bovin B.taurus (NM 174580), les 1080 bp de la séquence de porc S. scrofa (Q9TSV5), les 1080 bp de la séquence du singe P. troglodytes (Q7YR49), les 1413 bp de la séquence de l'homme H. sapiens pou5f1 (NM 002701), les 1141 bp de la séquence du rat R. rattus (XM 579282), les 3111 bp de la séquence de l'axolot A. mexicanum (AY542376.), les 1418 bp de la séquence du poisson zebra D. rerio (NM 131112), les 570 bp de la séquence du domaine POU du marsupial T.vulpecula (AY345973), les 2111 bp de la séquence du xénope X. laevis *Oct60* (M60075), les 2418 bp de la séquence du xénope X. Laevis *Oct*91 (M60077) et les 1998 bp de la séquence du xénope X. Laevis *Oct25* M60074),
on peut identifier que *1P06* est un orthologue des gènes *Pou5f1* des autres espèces, car la séquence identifiée est plus proche de la séquence de ces gènes que des séquences des gènes paralogues comprenant un domaine POU (Figures 4, 5 et 6).
La figure 6 montre que le cDNA *1P06* du poulet présente respectivement 51, 46 and 51 % d'homologie avec les orthologues bovin, murin et humain. Des pourcentages d'homologie de 35 à 38 % sont observés avec le gene *Spg* (Pou2) du poisson zèbre et des différents homologues de xénope.
La figure 7 montre les pourcentages d'homologie existant entre les différents paralogues connus et identifiés chez le poulet, et la séquence du cDNA *1P06 : 1P06* présente respectivement 14, 60, 62 et 61 % d'homologie avec les ADNc de *Oct-1, Factor I, Brn3.2* et *Oct6* (Barth et al., 1998, Levavasseur et al., 1998).

### EXEMPLE 5 : ETUDE DE LA PROTEINE PRODUIT DU GENE 1P06

### La protéine de fusion GFP-1P06 est une protéine nucléaire

Le vecteur commercial pE-GFP-C1 (Clontech) a été utilisé pour cloner différents cDNA en phase avec la GFP afin de visualiser leur localisation cellulaire après transfection.

Nous avons introduit dans ce vecteur les phase codantes des ADNc de *1P06, Nanog, Gcnf, Sox2, Lrh1, Sf1 et Gata4.*
Les plasmides pGFP-1P06, *Nanog, Gcnf,* etc ont été introduits de façon transitoire dans les cellules. Les cellules CESC sont ensemencées sur lamelle de verre stérile dans des puits à une densité moyenne de 15 000 à 20 000 cellules par cm2. Après quelques heures d'adhésion, le mélange de transfection comprenant 1 µg d'ADN linéaire, 3 µg de Fugene (Roche) dans 100 µl de milieu sans sérum est préparé, laissé pendant 10 min à température ambiante avant d'être déposé sur les cellules qui sont placées dans l'incubateur pendant une nuit dans les conditions normales de prolifération. Au jour 2, le mélange de transfection est retiré, les cellules sont lavées avec du PBS puis cultivées dans les conditions normales. Après 48h, les cellules sont lavées puis fixées avec un mélange en PBS de 0.5% glutaraldéhyde/ 1.5% formaldéhyde pendant 15 min à 4°C. Après lavage, les cellules sont incubées avec une solution de Hoescht (10 µM/ml) afin de visualiser les noyaux à l'abri de la lumière. Les lamelles sont montées en présence d'une substance garantissant le passage des UV (Gel Mont BioMedia) et l'observation de la fluorescence émise par la protéine de fusion est réalisée à l'aide d'un microscope à fluorescence.
Le nombre de cellules comptées est de n= 49 (GFP-T pour témoin), n=71 (GFP-1P06) et n=74 (GFP-Gata4). La figure 8 indique le pourcentage de cellules où la localisation observée de la fluorescence de la GFP est dans le noyau (N), dans le cytoplasme (C), ou dans le noyau et le cytoplasme (N+C). Cette figure montre que la protéine de fusion 1P06-GFP est principalement nucléaire.
Des résultats similaires ont été obtenus dans d'autres types cellulaires aviaires comme les fibroblastes embryonnaires de poulet (résultats non montrés).

Par ailleurs, des analyses par la technique du « band-shift » ont permis de démontrer que la protéine 1P06 était capable de reconnaître et de se fixer sur la séquence consensus Oct-4 de l'ADN.
En conclusion, la protéine 1P06 du poulet est une protéine à localisation nucléaire, qui présente une activité de fixation sur l'ADN.

### EXEMPLE 6 : SUREXPRESSION DU GENE 1P06

Afin d'évaluer l'impact de la surexpression du gène *1P06* dans la physiologie des cellules CESC, les cellules transfectées avec les constructions pGFP-1P06 et d'autres protéines de fusion telles que GFP-SOX2, GFP-NANOG, GFP- GATA4, ont été analysées d'un point de vue morphologique.
La figure 9 présente les résultats obtenus. Le nombre total de cellules comptées est de n= 51 (GFP-T témoin), n=93 (GFP-1P06) et n=77 (GFP-Gata4).
La plupart des cellules transfectées ne semblent pas présenter de modification majeure dans leur morphologie. Une exception est observée avec la construction pGFP-1P06, pour laquelle on observe une proportion élevée de forme nucléaire altérée, avec notamment des formes condensées du noyau, dans environ 20% des cas. Le pourcentage de cellules présentant une chromatine déstructurée est bien plus important dans ces cellules transfectées avec la construction GFP-1P06. Ceci pourrait indiquer un phénomène d'apoptose. Le temps de la transfection étant relativement court (<72h), il est envisageable de ne pas être en mesure de pouvoir identifier de plus grandes variations morphologiques et phénotypiques dans ces conditions.
Notre hypothèse est que la surexpression de la construction GFP-1P06 est toxique pour les cellules CESC. Une construction GFP-1P06-ERT2 a été réalisée afin de rendre conditionnelle la localisation nucléaire de la protéine fusion. Si la localisation nucléaire de la construction témoin GFP-ERT2 semble dépendante de la présence de 4-hydroxy-tamoxifène dans le milieu de culture, comme cela est attendue, il n'a pas été possible de détecter un changement majeur de localisation de la protéine fusion GFP-1P06-ERT2 en présence ou en absence de 4-hydroxy-tamoxifène. La présence de la GFP reste majoritairement nucléaire, quelles que soient les conditions. Il est à supposer que le signal de localisation nucléaire du produit 1P06 est trop fort pour être équilibré par la seule présence de la molécule chaperone qui se lie à la partie ERT2 en absence de 4-hydroxy tamoxifene. La même observation a été réalisée avec le mutant 1P06mut, dont la mutation affecte le signal de localisation putatif nls, pour lequel on ne parvient pas à obtenir une signalisation cytoplasmique majeure (Pan et al., 2004).

### EXEMPLE DE REFERENCE 7 : CLONAGE DU PROMOTEUR DU GENE NANOG

Le clonage du promoteur du gène *Nanog* permet de démontrer la présence de sites spécifiques de fixation de facteurs de transcription susceptibles de réguler l'expression de ces gènes dans les cellules souches embryonnaires et germinales aviaires.
Dans les expériences de transfection, les résultats suivants ont été obtenus avec les constructions du promoteur de *Nanog :* ce promoteur est actif exclusivement dans les cellules prolifératives, non différenciées. Ainsi, des transfections transitoires dans les cellules CESC non différenciées et induites en différenciation permettent de montrer que seules les cellules non différenciées sont capables de transactiver ce promoteur. Ainsi des cellules transfectées avec la construction p2000Nanog-GFP montrent une expression de la GFP dans 42% des cellules et seulement dans 7% des cellules quand celles-ci sont traitées pendant 48 h par 10⁻⁷M d'acide rétinoïque.

## Revendications

1. Procédé de préparation de cellules aviaires différenciées à partir de cellules souches aviaires cultivées dans un milieu de culture approprié, **caractérisé en ce qu'**il comprend une étape d'induction de la différenciation des cellules souches par inhibition de l'expression ou de l'activité du gène *1P06* de la SEQ ID NO.1 exprimé dans lesdites cellules souches.

2. Procédé selon la revendication 1, **caractérisé en ce que** l'inhibition de l'expression ou de l'activité du gène est réalisée au moyen d'au moins un ARN interférent, de préférence choisi parmi un ARN antisens, un ARN double brin, un «small interfering » ARN, un « small hairpin » ARN ou un ARN ribosomal.

3. Procédé selon la revendication 2, **caractérisé en ce que** la molécule d'acide nucléique codant pour l'ARN interférent présente la séquence SEQ ID N°3.

4. Procédé selon l'une des revendications 2 à 3, **caractérisé en ce que** la molécule d'acide nucléique codant pour l'ARN interférent est intégrée dans un vecteur d'expression, sous le contrôle d'un promoteur permettant l'expression dudit ARN interférent dans une cellule hôte.

5. Procédé selon la revendication 4, **caractérisé en ce que** le promoteur est un promoteur inductible.

6. Procédé selon la revendication 4, **caractérisé en ce que** le vecteur d'expression comprenant la molécule d'acide nucléique codant pour l'ARN interférent est intégré dans une cellule hôte.

7. Procédé selon l'une des revendications 1 à 6, **caractérisé en ce que** les cellules souches subissent au moins une étape d'activation ou d'inhibition de gènes spécifiques, dans le but d'obtenir une différenciation spécifique desdites cellules.

8. Procédé selon l'une des revendications 1 à 7, **caractérisé en ce que** les cellules souches sont cultivées dans des conditions appropriées pour obtenir une différenciation spécifique desdites cellules.

9. Procédé selon l'une des revendications 1 à 8, **caractérisé en ce qu'**il comprend une étape de sélection et destruction des cellules non-induites en différentiation.

10. Molécule d'acide nucléique codant pour un ARN interférent tel que défini dans la revendication 3.

11. Vecteur d'expression comprenant une molécule d'acide nucléique selon la revendication 10, placée sous le contrôle d'un promoteur permettant l'expression dudit ARN interférent dans une cellule hôte, de préférence sous le contrôle d'un promoteur inductible.

12. Cellules souches aviaires transformées avec un vecteur d'expression selon la revendication 11.

## Claims

1. Process for preparing differentiated avian cells from avian stem cells cultivated in an appropriate culture medium, **characterised in that** it includes a step of inducing the differentiation of the stem cells by inhibiting the expression or activity of the *1P06* gene of the SEQ ID No. 1 expressed in said stem cells.

2. Process according to claim 1, **characterised in that** the inhibition of the expression or activity of the gene is performed by use of at least one interfering RNA preferably chosen from an antisense RNA, a double-stranded RNA, a "small interfering" RNA, a "small hairpin" RNA or a ribosomal RNA.

3. Process according to claim 2, **characterised in that** the nucleic acid molecule coding for the interfering RNA has the sequence SEQ ID No. 3.

4. Process according to one of claims 2 to 3, **characterised in that** the nucleic acid molecule coding for the interfering RNA is integrated into an expression vector, under the control of a promoter enabling the expression of said interfering RNA in a host cell.

5. Process according to claim 4, **characterised in that** the promoter is an inducible promoter.

6. Process according to claim 4, **characterised in that** the expression vector including the nucleic acid molecule coding for the interfering RNA is integrated into a host cell.

7. Process according to one of claims 1 to 6, **characterised in that** the stem cells undergo at least one step of activating or inhibiting specific genes, for the purpose of obtaining a specific differentiation of said cells.

8. Process according to one of claims 1 to 7, **characterised in that** the stem cells are cultivated under conditions suitable for obtaining a specific differentiation of said cells.

9. Process according to one of claims 1 to 8, **characterised in that** it includes a step of selecting and destroying the cells that were not induced into differentiation.

10. Nucleic acid molecule coding for an interfering RNA as defined in claim 3.

11. Expression vector including a nucleic acid molecule according to claim 10, placed under the control of a promoter enabling the expression of said interfering RNA in a host cell, preferably under the control of an inducible promoter.

12. Avian stem cells transformed with an expression vector according to claim 11.

## Patentansprüche

1. Verfahren zur Herstellung differenzierter Vogelzellen aus Vogel-Stammzellen, kultiviert in einem geeigneten Kulturmedium, **dadurch gekennzeichnet, dass** es einen Schritt der Induktion der Differenzierung der Stammzellen durch Hemmung der Expression oder der Aktivität des Gens IP06 der SEQ ID N° 1, exprimiert in den Stammzellen, umfasst.

2. Verfahren nach Anspruch 1, **dadurch gekennzeichnet, dass** die Hemmung der Expression oder der Aktivität des Gens mithilfe mindestens einer Interferenz-RNA, vorzugsweise ausgewählt aus einer Gegensinn-RNA, einer Doppelstrang-RNA, einer "small interfering" RNA, einer "small hairpin" RNA oder einer ribosomalen RNA, durchgeführt wird.

3. Verfahren nach Anspruch 2, **dadurch gekennzeichnet, dass** das Nukleinsäuremolekül, codierend für die Interferenz-RNA, die Sequenz SEQ ID Nr. 3 aufweist.

4. Verfahren nach einem der Ansprüche 2 bis 3, **dadurch gekennzeichnet, dass** das Nukleinsäuremolekül, codierend für die Interferenz-RNA, in einen Expressionsvektor integriert ist, unter Kontrolle eines Promotors, der die Expression der Interferenz-RNA in einer Wirtszelle ermöglicht.

5. Verfahren nach Anspruch 4, **dadurch gekennzeichnet, dass** der Promotor ein induzierbarer Promotor ist.

6. Verfahren nach Anspruch 4, **dadurch gekennzeichnet, dass** der Expressionsvektor, umfassend das Nukleinsäuremolekül, codierend für die Interferenz-RNA, in eine Wirtszelle integriert ist.

7. Verfahren nach einem der Ansprüche 1 bis 6, **dadurch gekennzeichnet, dass** die Stammzellen mindestens einen Schritt des Aktivierens oder des Hemmens von spezifischen Genen durchlaufen, mit dem Ziel, eine spezifische Differenzierung der Zellen zu erhalten.

8. Verfahren nach einem der Ansprüche 1 bis 7, **dadurch gekennzeichnet, dass** die Stammzellen unter geeigneten Bedingungen kultiviert werden, um eine spezifische Differenzierung der Zellen zu erhalten.

9. Verfahren nach einem der Ansprüche 1 bis 8, **dadurch gekennzeichnet, dass** es einen Schritt des Auswählens und Zerstörens der nicht zur Differenzierung induzierten Zellen umfasst.

10. Nukleinsäuremolekül, codierend die Interferenz-RNA, wie in Anspruch 3 definiert.

11. Expressionsvektor, umfassend ein Nukleinsäuremolekül nach Anspruch 10, unter Kontrolle eines Promotors gestellt, der die Expression der Interferenz-RNA in einer Wirtszelle ermöglicht, vorzugsweise unter Kontrolle eines induzierbaren Promotors.

12. Vogel-Stammzellen, umgewandelt mit einem Expressionsvektor nach Anspruch 11.
